# EUROPÄISCHE PATENTSCHRIFT

(11) **EP 4 605 455 B1**
(45) Veröffentlichungstag und Bekanntmachung des Hinweises auf die Patenterteilung: **02.12.2026**
(21) Anmeldenummer: 22808663.3
(22) Anmeldetag: 19.10.2022
(51) Int. Cl.: C08J 11/24, C07C 67/03, C07C 69/82

(54) **VERBESSERTES VERFAHREN ZUR DEPOLYMERISIERUNG VON POLYETHYLENTEREPHTHALAT**
IMPROVED METHOD FOR THE DEPOLYMERISATION OF POLYETHYLENE TEREPHTHALATE
PROCÉDÉ AMÉLIORÉ DE DÉPOLYMÉRISATION DU POLYÉTHYLÈNE TÉRÉPHTALATE

(43) Veröffentlichungstag der Anmeldung: 27.08.2025
(73) Patentinhaber: Evonik Operations GmbH, 45128 Essen (DE)
(72) Erfinder: REINSBERG, Philip Heinrich, 53359 Rheinbach (DE); ZANDER, Christian, 63452 Hanau (DE); RUWWE, Johannes, 63457 Hanau (DE); BLUM, Adrian, 50354 Huerth (DE)
(74) Vertreter: Evonik Patent Association
(86) Internationale Anmeldenummer: PCT/EP2022/079049
(87) Internationale Veröffentlichungsnummer: WO 2024/083324

(56) Entgegenhaltungen:
- EP-A1- 0 723 951
- EP-A1- 1 997 794
- KR-B1- 101 888 612

## Beschreibung

Die vorliegende Erfindung betrifft ein Verfahren zur Depolymerisierung von Polyethylenterephthalat (= **"PET"**), in welchem **PET** in einer Mischung umfassend Glykol und M_{A}OR zu Bis-(2-hydroxyethyl)-terephthalat (= **"BHET";** CAS-Nr.: 959-26-2) umgesetzt wird, wobei M_{A} ein Alkalimetall ausgewählt aus Natrium, Kalium, Lithium ist, und wobei R ein Alkylrest mit 1 bis 6 Kohlenstoffatomen ist. M_{A}OR wird dabei durch Reaktivdestillation erhalten.

Das erfindungsgemäße Verfahren zeichnet sich dadurch aus, dass der Anteil der unerwünschten Spaltprodukte Mono-(2-hydroxyethyl)-terephthalat (= **"MHET")** und Terephthalat (= **"TS"**), bezogen auf den Anteil an **BHET,** besonders niedrig ist. Dadurch liefert das erfindungsgemäße Verfahren eine hohe Ausbeute an **BHET,** welches direkt für die erneute **PET**-Herstellung eingesetzt werden kann.

Die vorliegende Erfindung betrifft somit auch ein Verfahren zum Recycling von **PET,** in dem das im Verfahren zur Depolymerisierung von **PET** erhaltene **BHET,** gegebenenfalls nach weiterer Reinigung, wieder zu **PET** polymerisiert wird.

### Hintergrund der Erfindung

Polyethylenterephthalat (= **"PET")** ist einer der bedeutendsten Kunststoffe, der in Textilfasern, als Folien und als Material für Kunststoffflaschen verwendet wird. Allein 2007 lag die in Kunststoffflaschen verwendete Menge bei ~ 10⁷ t (W. Caseri, Polyethylenterephthalate, RD-16-03258 (2009) in F. Böckler, B. Dill, G. Eisenbrand, F. Faupel, B. Fugmann, T. Gamse, R. Matissek, G. Pohnert, A. Rühling, S. Schmidt, G. Sprenger, RÖMPP [Online], Stuttgart, Georg Thieme Verlag, Januar 2022).

Aufgrund seiner Haltbarkeit und die auf **PET** zurückzuführenden Müllmengen stellt es eine der größten ökologischen Herausforderungen der Gegenwart dar. Die Lösung dieses Problems liegt in der Vermeidung und in der effizienten Wiederverwertung von **PET.**

Im Stand der Technik werden mehrere Verfahren zur Spaltung von **PET** vorgeschlagen. GB 784,248 A beschreibt die Methanolyse von **PET.**

Hydrolytische Verfahren zur Depolymerisierung von **PET** beschreiben JP 2000-309663 A, US 4,355,175 A und T. Yoshioka, N. Okayama, A. Okuwaki, Ind. Eng. Chem. Res. 1998, 37, 336 - 340.

Die Umsetzung von **PET** mit Glykol wird in der EP 0723951 A1, der US 3,222,299 A, der WO 2020/002999 A2, von S.R. Shukla, A.M. Harad, Journal of Applied Polymer Science 2005, 97, 513 - 517 (im Folgenden "Shukla & Harad") und von N.D. Pingale, S.R. Shukla, European Polymer Journal 2008, 44, 4151 - 4156 beschrieben.

Shukla & Harad beschreiben, dass bei der **PET**-Glykolyse Bis-(2-hydroxyethyl)-terephthalat (= **"BHET")** entsteht. Dieses Spaltprodukt kann gleichzeitig als Edukt zur Herstellung neuen **PETs** eingesetzt werden. Demgegenüber sind bestimmte Nebenprodukte wie der Monoester Mono-(2-hydroxyethyl)-terephthalat (= **"MHET")** oder die freie Terephthalsäure bzw. das entsprechende Carboxylat, Terephthalat, (= **"TS**") nachteilig, da diese nicht direkt als Edukte zur Herstellung neuen **PETs** eingesetzt werden können.

Es besteht demnach ein Interesse an Verfahren zur Depolymerisierung von **PET,** bei dem ein möglichst hoher Anteil an **BHET** unter den Spaltprodukten erhalten wird, während der Anteil an unerwünschten Nebenprodukten wie **MHET** und **TS** möglichst gering sein soll.

Die Aufgabe der vorliegenden Erfindung bestand darin, ein solches Verfahren zur Verfügung zu stellen.

### Kurzbeschreibung der Erfindung

Es wurde überraschend gefunden, dass bei Umsetzung des **PETs** in Glykol mit durch Reaktivdestillation erhaltenem M_{A}OR ein, auf **BHET** bezogen, geringerer Anteil an unerwünschten Nebenprodukten **MHET** und **TS** erhalten wird als in herkömmlichen Verfahren.

Es wurde so überraschend ein Verfahren gefunden, das die erfindungsgemäße Aufgabe löst.

Die vorliegende Erfindung betrifft demnach ein Verfahren zur Depolymerisierung von Polyethylenterephthalat **PET,** umfassend die folgenden Schritte:
(a) M_{A}OH und ROH werden in einer Reaktivdestillation zu M_{A}OR umgesetzt.

Dabei ist M_{A} ein Alkalimetall ausgewählt aus Natrium, Kalium, Lithium, insbesondere ein Alkalimetall ausgewählt aus Natrium, Kalium und bevorzugt M_{A} = Natrium.

R ist ein Alkylrest mit 1 bis 6, insbesondere 1 bis 5, bevorzugt 1 bis 4, bevorzugter 1 bis 3 Kohlenstoffatomen. Noch bevorzugter ist R Methyl oder Ethyl. Am bevorzugtesten ist R = Methyl.

(b) **PET** wird in einer Mischung umfassend Glykol und mindestens einen Teil des in Schritt (a) erhaltenen M_{A}OR zu Bis-(2-hydroxyethyl)-terephthalat **BHET** umgesetzt.

In einem weiteren Aspekt betrifft die vorliegende Erfindung ein Verfahren zum Recycling von **PET,** in welchem in einem Schritt (ζ) das im erfindungsgemäßen Verfahren zur Depolymerisierung erhaltene **BHET** zu **PET** polymerisiert wird.

### Abbildung

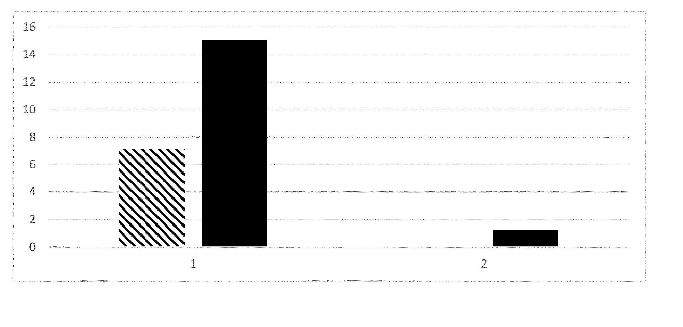

Die Abbildung zeigt den Vergleich des Gehalts von Mono-(2-hydroxyethyl)-terephthalsäure **("MHET";** "1") und Terephthalsäure (**"TS"**; "2") bei der Depolymerisierung mit gemäß dem erfindungsgemäßen Verfahren erhaltenem Natriummethylat sowie mit gemäß herkömmlichen Verfahren erhaltenem Natriummethylat. Der Gehalt des jeweiligen Nebenprodukts wird dabei bezogen auf den Gehalt an **BHET** angegeben (in molaren Prozent).

Der jeweilige Gehalt von **MHET** und **TS** im Reaktoraustrag bei der Depolymerisierung von **PET** gemäß dem erfinderischen Beispiel **E1,** in dem das für die Depolymerisierung verwendete Natriummethylat durch Reaktivdestillation erhalten wurde, wird jeweils durch schraffierte Balken ("\\\\\") angezeigt. Es wurde nur ein signifikanter Anteil von **MHET** festgestellt, weshalb bei "2" kein schraffierter Balken gezeigt ist.

Die schwarzen Balken zeigen den jeweiligen Gehalt von **MHET** und **TS** im Reaktoraustrag bei der Depolymerisierung von **PET** gemäß dem Vergleichsbeispiel **V1,** in dem das für die Depolymerisierung verwendete Natriummethylat durch Mischen von NaOH und Methanol im Reaktor erhalten wurde.

### Detaillierte Beschreibung der Erfindung

Es wurde nun überraschend gefunden, dass der Anteil an erwünschtem Spaltprodukt **BHET** bei der Glykolyse von **PET** gegenüber dem Anteil an unerwünschten Spaltprodukten **TS** und **MHET** erhöht ist, wenn die Spaltung des **PET** in einer Mischung aus Glykol und durch Reaktivdestillation erhaltenem M_{A}OR durchgeführt wird.

Das erfindungsgemäße Verfahren ist somit dem Verfahren des Standes der Technik überlegen, in welchen z.B. die Spaltung in einer Mischung durchgeführt wird, welche durch Auflösen der Alkalimetallhydroxide in Glykol und ROH erhalten wurde.

### 1. Schritt (a): Herstellung von M_{A}OR durch Reaktivdestillation

Das im erfindungsgemäßen Verfahren eingesetzte Alkalimetallalkoholat M_{A}OR wird erfindungsgemäß durch Umsetzung von M_{A}OH und ROH mittels Reaktivdestillation erhalten.

M_{A} ist ein Alkalimetall, welches aus Lithium, Natrium, Kalium, insbesondere aus Natrium, Kalium ausgewählt ist. M_{A} ist bevorzugt Natrium.

R ist ein Alkylrest mit 1 bis 6 Kohlenstoffatomen, insbesondere ein Alkylrest mit 1 bis 5 Kohlenstoffatomen, bevorzugt ein Alkylrest mit 1 bis 4 Kohlenstoffatomen, bevorzugter ein Alkylrest mit 1 bis 3 Kohlenstoffatomen. Noch bevorzugter ist R aus Ethyl, Methyl ausgewählt. Am bevorzugtesten ist R = Methyl.

Ein Alkylrest mit 1 bis 6 Kohlenstoffatomen ist insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, bevorzugt ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, *n*-Hexyl, noch bevorzugter ausgewählt aus der Gruppe bestehend aus Methyl, Ethyl, *n*-Propyl, *iso*-Propyl, *n*-Butyl, *n*-Pentyl, *n*-Hexyl.

Im Sinne der Erfindung ist ein Alkylrest mit 1 bis 5 Kohlenstoffatomen insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, bevorzugt ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, noch bevorzugter ausgewählt aus der Gruppe bestehend aus Methyl, Ethyl, *n*-Propyl, *iso*-Propyl, *n*-Butyl, *n*-Pentyl.

Im Sinne der Erfindung ist ein Alkylrest mit 1 bis 4 Kohlenstoffatomen insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, bevorzugt ausgewählt aus der Gruppe bestehend aus Methyl, Ethyl, *n*-Propyl, *iso*-Propyl, *n*-Butyl, *tert*-Butyl, noch bevorzugter ausgewählt aus der Gruppe bestehend aus Methyl, Ethyl, *n*-Propyl, *iso*-Propyl, *n*-Butyl.

Im Sinne der Erfindung ist ein Alkylrest mit 1 bis 3 Kohlenstoffatomen insbesondere ausgewählt aus der Gruppe bestehend aus Methyl, Ethyl, *n*-Propyl, *iso*-Propyl, bevorzugt ausgewählt aus der Gruppe bestehend aus Methyl, Ethyl, *iso*-Propyl.

Unter "Glykol" wird im Sinne der Erfindung 1,2-Ethylendiol mit der chemischen Formel HO-CH₂-CH₂-OH (CAS-Nr. 107-21-1) verstanden.

Die Reaktivdestillation zur Herstellung von Alkalimetallalkoholaten ist ein wichtiger industrieller Prozess, da Alkalimetallalkoholate als starke Basen in der Synthese zahlreicher Chemikalien, z.B. bei der Herstellung von Pharma- oder Agrowirkstoffen, und als Katalysatoren in Umesterungs- und Amidierungsreaktionen eingesetzt werden.

Alkalimetallalkoholate (MOR) werden mittels Reaktivdestillation typischerweise in einer Gegenstromdestillationskolonne aus Alkalimetallhydroxiden (MOH) und Alkoholen (ROH) hergestellt, wobei das gemäß folgender Reaktion <1> entstehende Reaktionswasser mit dem Destillat entfernt wird. MOH + ROH 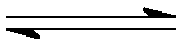 MOR + H₂O.

Ein solches Verfahrensprinzip ist beispielsweise in der US 2,877,274 A beschrieben, wobei wässrige Alkalimetallhydroxid-Lösung und gasförmiges Methanol im Gegenstrom in einer Rektifikationskolonne gefahren werden. In prinzipiell nicht veränderter Form wird dieses Verfahren in der WO 01/42178 A1 erneut beschrieben.

Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1, der WO 2021/148174 A1 und der WO 2021/148175 A1.

Ähnliche Verfahren, bei denen jedoch zusätzlich noch ein Schleppmittel, wie beispielsweise Benzol, eingesetzt wird, sind in der GB 377,631 A und der US 1,910,331 A beschrieben.

Entsprechend beschreibt die DE 96 89 03 C ein Verfahren zur kontinuierlichen Herstellung von Alkalimetallalkoholaten in einer Reaktionskolonne, wobei das am Kopf entnommene Wasser-Alkohol-Gemisch kondensiert und anschließend einer Phasentrennung unterworfen wird. Die wässrige Phase wird hierbei verworfen und die alkoholische Phase wird zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt.

In einer bevorzugten Ausführungsform des erfindungsgemäßen Verfahrens wird in Schritt (a) ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt, wobei am unteren Ende von **RR_{A}** ein Sumpfproduktstrom **S_{AP}** umfassend M_{A}OR und ROH entnommen wird und wobei am oberen Ende von **RR_{A}** ein Brüdenstrom **S_{AB}** umfassend Wasser und gegebenenfalls ROH entnommen wird.

Als "Reaktivrektifikationskolonne" wird erfindungsgemäß eine Rektifikationskolonne definiert, in der zumindest in einigen Teilen die Umsetzung nach Schritt (a) des erfindungsgemäßen Verfahrens abläuft. Sie kann auch abgekürzt als "Reaktionskolonne" bezeichnet werden.

Der Eduktstrom **S_{AE1}** umfasst ROH. In einer bevorzugten Ausführungsform liegt der Massenanteil von ROH in **S_{AE1}** bei ≥ 95 Gew.-%, noch bevorzugter bei ≥ 99.5 Gew.-%, wobei **S_{AE1}** ansonsten insbesondere Wasser aufweist.

Das in der bevorzugten Ausführungsform des erfindungsgemäßen Verfahrens als Eduktstrom **S_{AE1}** eingesetzte ROH kann auch handelsüblicher Alkohol ROH mit einem Massenanteil an ROH von mehr als 99.5 Gew.-% und einem Massenanteil an Wasser von bis zu 0.03 Gew.-% sein.

Der Eduktstrom **S_{AE1}** wird in einer besonderen Ausführungsform der vorliegenden Erfindung dampfförmig zur Reaktivrektifikationskolonne **RR_{A}** zugegeben.

In einer alternativen, bevorzugten Ausführungsform des erfindungsgemäßen Verfahrens wird ROH vor Schritt (a) im Sumpf der Reaktivrektifikationskolonne **RR_{A}** vorgelegt, und dann im Schritt (a) zum Sieden erhitzt wird, wodurch in der Reaktivrektifikationskolonne **RR_{A}** ein konstanter Eduktstrom **S_{AE1}** erzeugt wird. Gegebenenfalls wird dann während der Durchführung des Schrittes (a) ROH in den Sumpf der Reaktivrektifikationskolonne **RR_{A}** nachgefüllt.

Der Eduktstrom **S_{AE2}** umfasst M_{A}OH. In einer bevorzugten Ausführungsform umfasst S_{AE2} neben M_{A}OH mindestens eine weitere Verbindung ausgewählt aus Wasser, ROH. Noch bevorzugter umfasst S_{AE2} neben M_{A}OH Wasser, dann handelt es sich bei **S_{AE2}** um eine wässrige Lösung von M_{A}OH.

Wenn der Eduktstrom **S_{AE2}** M_{A}OH und Wasser umfasst, liegt der Massenanteil von M_{A}OH, bezogen auf das Gesamtgewicht der wässrigen Lösung, welche **S_{AE2}** bildet, insbesondere im Bereich von 10 bis 75 Gew.-%, bevorzugt im Bereich von 15 bis 54 Gew.-%, bevorzugter im Bereich von 30 bis 53 Gew.-%, noch bevorzugter im Bereich von 40 bis 52 Gew.-% und am bevorzugtesten bei 50 Gew.-%.

Der Schritt (a) des erfindungsgemäßen Verfahrens wird bevorzugt in einer Reaktivrektifikationskolonne (oder "Reaktionskolonne") **RR_{A}** durchgeführt.

Bevorzugt enthält die Reaktionskolonne **RR_{A}** Einbauten. Geeignete Einbauten sind zum Beispiel Böden, strukturierte Packungen oder unstrukturierte Packungen. Wenn die Reaktionskolonne **RR_{A}** Böden enthält, so sind Glockenböden, Ventilböden, Tunnelböden, Thormann-Böden, Kreuzschlitzglockenböden oder Siebböden geeignet. Wenn die Reaktionskolonne **RR_{A}** Böden enthält, so werden vorzugsweise solche Böden gewählt, bei denen maximal 5 Gew.-%, bevorzugt weniger als 1 Gew.-% der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf das Doppelte des Wertes, der üblicherweise eingestellt wird, erhöhen. Bei Einsatz von Siebböden ist es besonders günstig, die Durchmesser der Bodenöffnungen zu verringern und die Zahl der Öffnungen beizubehalten oder noch zu vergrößern.

Bei Einsatz von strukturierten oder unstrukturierten Packungen sind strukturierte Packungen im Hinblick auf die gleichmäßige Verteilung der Flüssigkeit bevorzugt.

Der Schritt (a) des erfindungsgemäßen Verfahrens kann sowohl kontinuierlich als auch diskontinuierlich erfolgen. Bevorzugt erfolgt er kontinuierlich.

"Umsetzung eines Eduktstroms **S_{AE1}** umfassend ROH mit einem Eduktstrom **S_{AE2}** umfassend M_{A}OH im Gegenstrom in einer Reaktivrektifikationskolonne **RR_{A}"** wird in einer erfindungsgemäßen Ausführungsform insbesondere dadurch gewährleistet, dass die Zulaufstelle mindestens eines Teils des Eduktstroms **S_{AE1}** umfassend ROH an der Reaktionskolonne **RR_{A}** unterhalb der Zulaufstelle des Eduktstroms **S_{AE2}** umfassend M_{A}OH liegt.

Die Reaktionskolonne **RR_{A}** umfasst in dieser Ausführungsform vorzugsweise mindestens 2, insbesondere 15 bis 40 theoretische Stufen zwischen der Zulaufstelle des Eduktstroms **S_{AE1}** und der Zulaufstelle des Eduktstroms **S_{AE2}.**

Die Reaktionskolonne **RR_{A}** kann als reine Abtriebskolonne betrieben werden. Dann wird im unteren Bereich der Reaktionskolonne **RR_{A}** dampfförmig der Eduktstrom **S_{AE1}** umfassend ROH zugeführt.

Optional wird ein Teil des Eduktstroms **S_{AE1}** umfassend ROH zwar unterhalb der Zulaufstelle des Eduktstroms S_{AE2} umfassend Alkalilauge M_{A}OH, aber dennoch am oberen Ende oder im Bereich des oberen Endes der Reaktionskolonne **RR_{A}** dampfförmig zugegeben. Dadurch können die Abmessungen im unteren Bereich der Reaktionskolonne **RR_{A}** verringert werden. Wenn ein Teil des Eduktstroms **S_{AE1}** umfassend ROH am oberen Ende oder im Bereich des oberen Endes der Reaktionskolonne **RR_{A}** insbesondere dampfförmig zugegeben wird, so wird bevorzugt nur eine Teilmenge von 10 bis 70 Gew.-%, bevorzugt von 30 bis 50 Gew.-% (jeweils bezogen auf die Gesamtmenge des eingesetzten Glykols) am unteren Ende der Reaktionskolonne **RR_{A}** eingespeist und die restliche Teilmenge in einem Einzelstrom oder auf mehrere Teilströme verteilt, bevorzugt 1 bis 10 theoretische Stufen, besonders bevorzugt 1 bis 3 theoretische Stufen unterhalb der Zulaufstelle des Eduktstroms **S_{AE2}** umfassend M_{A}OH dampfförmig zugegeben.

In einer alternativen Ausführungsform des Schrittes (a) des erfindungsgemäßen Verfahrens wird "Umsetzung eines Eduktstroms **S_{AE1}** umfassend ROH mit einem Eduktstrom **S_{AE2}** umfassend M_{A}OH im Gegenstrom in einer Reaktivrektifikationskolonne **RR_{A}"** insbesondere dadurch gewährleistet, dass sich ROH im Sumpf der Reaktivrektifikationskolonne **RR_{A}** befindet und die Zulaufstelle des Eduktstroms **S_{AE2}** umfassend M_{A}OH oberhalb des Sumpfes liegt. Während des Schrittes (a) des erfindungsgemäßen Verfahrens wird dann ROH im Sumpf von **RR_{A}** zum Sieden erhitzt und ein Eduktstrom **S_{AE1}** umfassend ROH erzeugt. **S_{AE1}** und **S_{AE2}** sind dann im Gegenstrom zueinander gerichtet.

In der Reaktionskolonne **RR_{A}** setzt sich dann der Eduktstrom **S_{AE1}** umfassend ROH mit dem Eduktstrom **S_{AE2}** umfassend M_{A}OH gemäß der vorstehend beschriebenen Reaktion **<1>** zu M_{A}OR und H₂O um, wobei, da es sich um eine Gleichgewichtsreaktion handelt, diese Produkte in Mischung mit den Edukten ROH und M_{A}OH vorliegen. Demnach wird in Schritt (a) ein Rohprodukt **RP_{A}** in der Reaktionskolonne **RR_{A}** erhalten, welches neben den Produkten M_{A}OR und Wasser auch noch ROH und M_{A}OH umfasst.

Am unteren Ende von **RR_{A}** erhält und entnimmt man dann den Sumpfproduktstrom **S_{AP}** umfassend ROH und M_{A}OR.

Am oberen Ende von **RR_{A},** bevorzugt am Kolonnenkopf von **RR_{A},** entnimmt man in einer bevorzugten Ausführungsform des erfindungsgemäßen Verfahrens, noch gegebenenfalls ROH enthaltenden Wasserstrom, vorstehend als "Brüdenstrom **S_{AB}** umfassend Wasser und gegebenenfalls ROH" bezeichnet.

Enthält der Brüdenstrom **S_{AB}** neben Wasser auch ROH, wird, bevorzugt destillativ, zum Beispiel in einer Rektifikationskolonne, ROH gewonnen. Mindestens ein Teil des bei der Destillation gewonnenen ROH kann in dieser Ausführungsform der Reaktionskolonne **RR_{A}** als Eduktstrom **S_{AE1}** wieder zugeführt werden.

In einer bevorzugten Ausführungsform wird, wenn **S_{AB}** Wasser und ROH umfasst, Wasser und ROH in einer Rektifikationskolonne **RD_{A}** mindestens teilweise voneinander getrennt (nachfolgend unter Punkt 2. beschrieben).

Die Menge des vom Eduktstrom **S_{AE1}** umfassten ROH wird vorzugsweise so gewählt, dass dieser gleichzeitig als Lösungsmittel für das im Sumpfproduktstrom **S_{AP}** erhaltene M_{A}OR dient. Vorzugsweise wird die Menge des ROH im Eduktstrom **S_{AE1}** so gewählt, dass im Sumpf der Reaktionskolonne die gewünschte Konzentration der M_{A}OR-Lösung vorliegt, die als Sumpfproduktstrom **S_{AP}** umfassend ROH und M_{A}OR entnommen wird.

In einer bevorzugten Ausführungsform des erfindungsgemäßen Verfahrens, und insbesondere in den Fällen, in denen **S_{AE2}** neben M_{A}OH auch Wasser umfasst, beträgt das Verhältnis des Gesamtgewichts (Massen; Einheit: kg) an als Eduktstrom **S_{AE1}** eingesetztem ROH zum Gesamtgewicht (Massen; Einheit: kg) an als Eduktstrom **S_{AE2}** eingesetzten M_{A}OH 1 : 1 bis 50 : 1, "Mit Rücklauf" bedeutet, dass der am oberen Ende der jeweiligen Kolonne, insbesondere der Reaktionskolonne **RR_{A},** entnommene Brüdenstrom **S_{AB}** umfassend Wasser und gegebenenfalls ROH nicht vollständig abgeführt wird. Der betreffende Brüdenstrom **S_{AB}** wird also mindestens teilweise, bevorzugt teilweise, wieder als Rücklauf der jeweiligen Kolonne, insbesondere der Reaktionskolonne **RR_{A},** zugeführt. In den Fällen, in denen ein solcher Rücklauf eingestellt wird, beträgt das Rücklaufverhältnis dabei bevorzugt 0.01 bis 1, bevorzugter 0.02 bis 0.9, noch bevorzugter 0.03 bis 0.34, besonderes bevorzugt 0.04 bis 0.27 und ganz besonders bevorzugt 0.05 bis 0.24, am bevorzugtesten 0.2.

Unter einem Rücklaufverhältnis wird allgemein und im Sinne dieser Erfindung das Verhältnis aus dem Anteil des aus der Kolonne entnommenen Massenstroms (kg/h), der in flüssiger Form oder gasförmiger Form von der jeweiligen Kolonne abgeführt wird zu dem Anteil dieses Massenstroms (kg/h), der wieder auf die Kolonne in flüssiger Form zurückgeführt wird (Rücklauf), verstanden.

Ein Rücklauf kann dadurch eingestellt werden, dass am Kopf der jeweiligen Kolonne ein Kondensator angebracht wird. Dazu wird insbesondere an der Reaktionskolonne **RR_{A}** ein Kondensator **K_{RRA}** angebracht. Im Kondensator **K_{RRA}** wird der Brüdenstrom **S_{AB}** mindestens teilweise kondensiert und der jeweiligen Kolonne, insbesondere der Reaktionskolonne **RR_{A}** wieder zugeführt.

In der Ausführungsform, in der an der Reaktionskolonne **RR_{A}** ein Rücklauf eingestellt wird, kann der in der bevorzugten Ausführungsform des erfindungsgemäßen Verfahrens als Eduktstrom **S_{AE2}** eingesetzte M_{A}OH auch mindestens teilweise mit dem Rücklaufstrom vermischt werden und die resultierende Mischung so der Reaktionskolonne **RR_{A}** zugeführt werden.

In einer bevorzugten Ausführungsform des erfindungsgemäßen Verfahrens wird Schritt (a) insbesondere bei destillativen Bedingungen durchgeführt, bei denen ROH refluxiert.

Der Schritt (a) wird insbesondere bei einer Temperatur im Bereich von 45 °C bis 150 °C, bevorzugt 47 °C bis 120 °C, bevorzugter 60 °C bis 110 °C, und bei einem Druck von 0.05 MPa (0.5 bar) abs. bis 4.0 MPa (40 bar) abs., bevorzugt im Bereich von 0.07 MPa (0.7 bar) abs. bis 0.5 MPa (5 bar) abs., bevorzugter im Bereich von 0.08 MPa (0.8 bar) abs. bis 0.4 MPa (4 bar) abs., bevorzugter im Bereich von 0.09 MPa (0.9 bar) abs. bis 0.35 MPa (3.5 bar) abs., noch bevorzugter bei 0.1 MPa (1.0 bar) abs. bis 0.3 MPa (3 bar) abs. durchgeführt.

Die Reaktionskolonne **RR_{A}** umfasst in einer bevorzugteren Ausführungsform mindestens einen Verdampfer, der insbesondere aus Zwischenverdampfern **V_{ZA}** und Sumpfverdampfern **V_{SA}** ausgewählt ist. Die Reaktionskolonne **RR_{A}** umfasst besonders bevorzugt mindestens einen Sumpfverdampfer **V_{SA}.**

Als "Zwischenverdampfer" **V_{Z}** werden erfindungsgemäß Verdampfer bezeichnet, die sich oberhalb des Sumpfs der jeweiligen Kolonne, insbesondere oberhalb des Sumpfs der Reaktionskolonne

Die Reaktionskolonne **RR_{A}** umfasst in einer bevorzugteren Ausführungsform mindestens einen Verdampfer, der insbesondere aus Zwischenverdampfern **V_{ZA}** und Sumpfverdampfern **V_{SA}** ausgewählt ist. Die Reaktionskolonne **RR_{A}** umfasst besonders bevorzugt mindestens einen Sumpfverdampfer **V_{SA}.**

Als "Zwischenverdampfer" **V_{Z}** werden erfindungsgemäß Verdampfer bezeichnet, die sich oberhalb des Sumpfs der jeweiligen Kolonne, insbesondere oberhalb des Sumpfs der Reaktionskolonne **RR_{A}** (dann als **"V_{ZA}"** bezeichnet) bzw. der in der bevorzugten Ausführungsform verwendeten und weiter unten näher beschriebenen Rektifikationskolonne **RD_{A}** (dann als **"V_{ZRD}"** bezeichnet) befinden. Im Falle von **RR_{A}** wird in ihnen insbesondere Rohprodukt **RP_{A}** verdampft, das der Kolonne als Seitenstrom **S_{ZAA}** entnommen wird.

Als "Sumpfverdampfer" **V_{S}** werden erfindungsgemäß Verdampfer bezeichnet, die den Sumpf der jeweiligen Kolonne, insbesondere den Sumpf der Reaktionskolonne **RR_{A}** bzw. den Sumpf der in der bevorzugten Ausführungsform verwendeten und weiter unten näher beschriebenen Rektifikationskolonne **RD_{A}** (dann als **"V_{SRD}"** oder **"V**_{**SRD**'}" bezeichnet) beheizen. Im Falle von **RR_{A}** wird in ihnen insbesondere mindestens ein Teil des Sumpfproduktstroms **S_{AP}** verdampft. Im Falle von **RD_{A}** wird in ihnen insbesondere Sumpfproduktstrom **S_{UA}** oder ein Teil von **S_{UA}**, **S_{UA1}**, verdampft.

Ein Verdampfer ist üblicherweise außerhalb der jeweiligen Reaktionskolonne oder Rektifikationskolonne angeordnet.

Geeignete Verdampfer, die als Zwischenverdampfer und Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Bei Einsatz eines Rohrbündelüberträgers kann der Wärmeträger entweder durch die Rohre strömen und das zu verdampfende Gemisch die Rohre umströmen oder aber der Wärmeträger umströmt die Rohre und das zu verdampfende Gemisch durchströmt die Rohre. Bei einem Fallfilmverdampfer wird das zu verdampfende Gemisch üblicherweise als dünner Film auf der Innenseite eines Rohres zugegeben und das Rohr wird von außen beheizt. Im Unterschied zu einem Fallfilmverdampfer ist in einem Dünnschichtverdampfer zusätzlich ein Rotor mit Wischern vorgesehen, der die zu verdampfende Flüssigkeit auf der Innenwand des Rohres zu einem dünnen Film verteilt.

Neben den genannten kann aber auch jede beliebige andere, dem Fachmann bekannte Verdampferbauart, die sich zum Einsatz an einer Rektifikationskolonne eignet, eingesetzt werden.

In der bevorzugten Ausführungsform des erfindungsgemäßen Verfahrens wird am unteren Ende der Reaktionskolonne **RR_{A}** ein Sumpfproduktstrom **S_{AP}** umfassend ROH und M_{A}OR als Sumpfproduktstrom entnommen.

In einer bevorzugten Ausführungsform des nachfolgend beschriebenen optionalen Schritts (a*) weist die Reaktionskolonne **RR_{A}** mindestens einen Sumpfverdampfer **V_{SA}** auf, über den der Sumpfproduktstrom **S_{AP}** dann teilweise geleitet wird und ROH aus diesem teilweise entfernt wird, wodurch ein Sumpfproduktstrom **S_{AP*}** mit einem gegenüber **S_{AP}** verringerten Massenanteil an ROH erhalten wird.

Insbesondere weist im erfindungsgemäßen Verfahren **S_{AP}** oder, falls mindestens ein Sumpfverdampfer **V_{SA}** eingesetzt wird, über den der Sumpfproduktstrom **S_{AP}** mindestens teilweise geleitet wird und ROH aus diesem mindestens teilweise entfernt wird, **S_{AP*},** einen Massenanteil von M_{A}OR in ROH im Bereich von 1 bis 50 Gew.-%, bevorzugt im Bereich von 5 bis 35 Gew.-%, bevorzugter im Bereich von 15 bis 35 Gew.-%, am bevorzugtesten im Bereich von 20 bis 35 Gew.-% auf, jeweils bezogen auf die Gesamtmasse von **S_{AP}** bzw. **S_{AP*}.**

Der Massenanteil an Restwasser in **S_{AP}** bzw. **S_{AP}*** liegt dabei bevorzugt bei < 1 Gew.-%, bevorzugt < 0.8 Gew.-%, bevorzugter < 0.5 Gew.-%, bezogen auf die Gesamtmasse von **S_{AP}** bzw. **S_{AP}***.

Der Massenanteil an Edukt M_{A}OH in **S_{AP}** bzw. **S_{AP*}** liegt dabei bevorzugt bei < 1 Gew.-%, bevorzugt < 0.8 Gew.-%, bevorzugter < 0.5 Gew.-%, bezogen auf die Gesamtmasse von **S_{AP}** bzw. **S_{AP}***.

Wir vorstehend beschrieben wird in einer bevorzugten Ausführungsform des erfindungsgemäßen Verfahrens am oberen Ende von **RR_{A}** ein Brüdenstrom **S_{AB}** umfassend Wasser und gegebenenfalls ROH entnommen.

### 2. Rektifikation des Brüdenstroms S_{AB} in einer Rektifikationskolonne R_{DA} (bevorzugt)

In einer bevorzugten Ausführungsform des erfindungsgemäßen Verfahrens werden, wenn der Brüdenstrom **S_{AB}** Wasser und ROH umfasst, diese (also das vom Brüdenstrom **S_{AB}** umfasste Wasser und das vom Brüdenstrom **S_{AB}** umfasste ROH) in einer Rektifikationskolonne **RD_{A}** mindestens teilweise voneinander getrennt.

Insbesondere wird **S_{AB}** umfassend Wasser und ROH in die Rektifikationskolonne **RD_{A}** geleitet und in **RD_{A}** in mindestens einen Strom **S₁** umfassend Wasser, und mindestens einen Strom **S₂** umfassend ROH aufgetrennt.

Es versteht sich von selbst, dass dabei das Verhältnis der Siedepunkte von Wasser und ROH bestimmt, welcher der beiden Ströme **S₁** (umfassend Wasser) und **S₂** (umfassend ROH) als Brüden- bzw. Sumpfstrom erhalten wird:
(i) Wenn ROH einen niedrigeren Siedepunkt als Wasser hat, was insbesondere der Fall ist für R = Alkylrest mit 1 bis 3, bevorzugt 1 bis 2, Kohlenstoffatomen ist, wird **S₂** (umfassend ROH) als Brüdenstrom am oberen Ende von **RD_{A}** erhalten und dort entnommen und **S₁** (umfassend Wasser) am unteren Ende von **RD_{A}** erhalten und dort entnommen;
(ii) Wenn Wasser einen niedrigeren Siedepunkt als ROH hat, was insbesondere der Fall ist für R = Alkylrest mit 5 bis 6 Kohlenstoffatomen, wird **S₁** (umfassend Wasser) als Brüdenstrom am oberen Ende von **RD_{A}** erhalten und dort entnommen und **S₂** (umfassend ROH) am unteren Ende von **RD_{A}** erhalten und dort entnommen.

Der Brüdenstrom **S_{AB}** kann dabei über eine oder mehrere Zulaufstellen in die Rektifikationskolonne **RD_{A}** geleitet werden. In den Ausführungsformen der vorliegenden Erfindung, in denen der Brüdenstrom **S_{AB}** als zwei oder mehrere voneinander getrennte Ströme in die Rektifikationskolonne **R_{DA}** geleitet wird, ist es vorteilhaft, wenn die Zulaufstellen der einzelnen Ströme im Wesentlichen auf der gleichen Höhe an der Rektifikationskolonne **RD_{A}** liegen.

Eine andere Bezeichnung für "oberes Ende einer Rektifikationskolonne" ist "Kopf".

Eine andere Bezeichnung für "unteres Ende einer Rektifikationskolonne" ist "Sumpf" oder "Fuß".

Als Rektifikationskolonne **RD_{A}** kann jede beliebige, dem Fachmann bekannte Rektifikationskolonne eingesetzt werden.

Bevorzugt enthält die Rektifikationskolonne **RD_{A}** Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne **RD_{A}** möglichst gering bleibt und somit die mechanische Leistung des Verdichters und die Temperatur des zu verdampfenden ROH/Wasser-Gemischs gering bleibt.

Wenn in der Rektifikationskolonne **RD_{A}** strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des Brüdenstroms **S_{AB}** und eine Packung unterhalb der Zulaufstelle des Brüdenstroms **S_{AB}.** Es können auch eine Packung oberhalb der Zulaufstelle des Brüdenstroms **S_{AB}** und mehrere Böden unterhalb der Zulaufstelle des Brüdenstroms **S_{AB}** vorgesehen sein. Wenn eine unstrukturierte Packung eingesetzt wird, beispielsweise eine Füllkörperpackung, so liegen die Füllkörper üblicherweise auf einem geeigneten Tragrost (z.B. Siebboden oder Gitterboden) auf.

In dieser bevorzugten Ausführungsform wird dann **S₁** oder **S₂** als Brüdenstrom am oberen Ende und **S₂** bzw. **S₁** als Sumpfstrom am unteren Ende der Rektifikationskolonne **RD_{A}** entnommen.

Der bevorzugte Massenanteil von Wasser in **S₁** ist ≥ 96.0 Gew.-%, bevorzugter ≥ 99.6 Gew.-%, noch bevorzugter ≥ 99.9 Gew.-%, wobei der Rest insbesondere ROH ist.

**S₂** umfasst ROH, wobei **S₂** bevorzugt < 1 Gew.-%, bevorzugter ≤ 5000 Gew.-ppm, noch bevorzugter ≤ 1000 Gew.-ppm, bevorzugter ≤ 100 Gew.-ppm Wasser aufweisen kann.

Die Entnahme mindestens einen Brüdenstroms am Kopf der Rektifikationskolonne **RD_{A}** bedeutet im Rahmen der vorliegenden Erfindung insbesondere, dass der mindestens eine Brüdenstrom als Kopfstrom oder als Seitenabzug oberhalb der Einbauten in der Rektifikationskolonne **RD_{A}** entnommen wird.

Die Entnahme des mindestens einen Stroms am Sumpf der Rektifikationskolonne **RD_{A}** bedeutet im Rahmen der vorliegenden Erfindung insbesondere, dass der mindestens ein Strom als Sumpfstrom oder am unteren Boden der Rektifikationskolonne **RD_{A}** entnommen wird.

Die Rektifikationskolonne **RD_{A}** wird mit oder ohne, vorzugsweise mit Rücklauf betrieben.

"Mit Rücklauf" bedeutet, dass der am oberen Ende der Rektifikationskolonne **RD_{A}** entnommene Brüdenstrom nicht vollständig abgeführt wird, sondern teilweise kondensiert und wieder der Rektifikationskolonne **RD_{A}** zugeführt wird. In den Fällen, in denen ein solcher Rücklauf eingestellt wird, beträgt das Rücklaufverhältnis dabei bevorzugt 0.01 bis 1, bevorzugter 0.02 bis 0.9, noch bevorzugter 0.03 bis 0.34, besonderes bevorzugt 0.04 bis 0.27 und ganz besonders bevorzugt 0.05 bis 0.24, am bevorzugtesten 0.2.

Ein Rücklauf kann dadurch eingestellt werden, dass am Kopf der Rektifikationskolonne **RD_{A}** ein Kondensator **K_{RD}** angebracht wird. In dem Kondensator **K_{RD}** wird der jeweilige Brüdenstrom **S_{OA}** teilweise kondensiert und der Rektifikationskolonne **RD_{A}** wieder zugeführt.

### 3. Optionaler Schritt (a*): Entfernung von ROH aus S_{AP}

In der bevorzugten Ausführungsform des Schritts (a) des erfindungsgemäßen Verfahrens, in welcher ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt wird,
und in welcher am unteren Ende von **RR_{A}** ein Sumpfproduktstrom **S_{AP}** umfassend M_{A}OR und ROH entnommen wird und wobei am oberen Ende von **RR_{A}** ein Brüdenstrom **S_{AB}** umfassend Wasser und gegebenenfalls ROH entnommen wird,
kann erfindungsgemäß in einem optionalen Schritt (a*) ROH mindestens teilweise aus **S_{AP}** entfernt werden, wodurch entweder eine Lösung **S_{AP}*** umfassend M_{A}OR und ROH, wobei **S_{AP}*** einen gegenüber **S_{AP}** verringerten Massenanteil an ROH aufweist, erhalten wird oder M_{A}OR als Feststoff **F*** erhalten wird.

Ob in dieser bevorzugten Ausführungsform (a*) die Lösung **S_{AP}*** oder der Feststoff F* erhalten wird, hängt davon ab, ob ROH teilweise oder im Wesentlichen vollständig aus **S_{AP}** entfernt wird.

Die mindestens teilweise Entfernung von ROH aus **S_{AP}** im optionalen Schritt (a*) kann gemäß dem Fachmann bekannten Verfahren durchgeführt werden. Beispielsweise kann, wie vorstehend beschrieben, die Reaktionskolonne **RR_{A}** mindestens einen Sumpfverdampfer **V_{SA}** aufweisen, über den der Sumpfproduktstrom **S_{AP}** dann teilweise geleitet wird und ROH aus diesem teilweise entfernt wird, wodurch ein Sumpfproduktstrom **S_{AP*}** mit einem gegenüber **S_{AP}** verringerten Gehalt an ROH erhalten wird.

Alternativ und bevorzugt kann ROH aus **S_{AP}** auch im Wesentlichen vollständig entfernt werden, z.B. in dem Fachmann bekannten Destillierapparaturen. Dann wird M_{A}OR als Feststoff **F*** erhalten.

Falls Schritt (a*) durchgeführt wird, liegt der Wassergehalt in **S_{AP}*** bzw. **F*** insbesondere bei < 1 Gew.-%, bevorzugt bei < 0.8 Gew.-%, bevorzugter < 0.5 Gew.-%, bezogen auf die Gesamtmasse von S_{AP}* bzw. **F*.**

### 4. Schritt (b): Umsetzen von PET zu BHET

In Schritt (b) des erfindungsgemäßen Verfahrens wird **PET** in einer Mischung umfassend Glykol und mindestens einen Teil des in Schritt (a) erhaltenen M_{A}OR zu **BHET** umgesetzt.

### 4.1 PET-Ausgangsmaterial

Als **PET,** welches im Schritt (b) des erfindungsgemäßen Verfahrens eingesetzt wird, kann jegliches **PET** eingesetzt werden, was depolymerisiert werden muss. Typischerweise fällt solches **PET** als Abfall an, insbesondere im Haushalt, in der Industrie, im Gesundheitssystem (z.B. Krankenhäusern, Arztpraxen) oder in der Landwirtschaft.

In einer Ausführungsform des erfindungsgemäßen Verfahrens liegt das damit zu depolymerisierende **PET** in Mischung mit anderen Kunststoffen, insbesondere mindestens einem Kunststoff ausgewählt aus Polyethylen **("PE")**, Polyvinylchlorid ("**PVC**") vor. Dies ist typischerweise der Fall, wenn im erfindungsgemäßen Verfahrens **PET** aus Kunststoffabfällen depolymerisiert werden soll. In dieser Ausführungsform wird das **PET** mindestens teilweise von den anderen Kunststoffen, bevorzugt durch Aussortieren, abgetrennt, bevor es dem Schritt (b) des erfindungsgemäßen Verfahrens unterworfen wird.

In einer Ausführungsform des erfindungsgemäßen Verfahrens wird das **PET** mindestens einem Vorbehandlungsschritt ausgesetzt.

Solche Vorbehandlungsschritte sind beispielsweise beschrieben in DE 10032899 C2.

Erfindungsgemäß wird das **PET** mindestens einem Vorbehandlungsschritt ausgewählt aus chemischem Vorbehandlungsschritt, Zerkleinerungsschritt unterworfen, bevor es in Schritt (b) eingesetzt wird.

In den Fällen, in denen das **PET** in Mischung mit anderen Kunststoffen vorliegt, wird das **PET** bevorzugt mindestens einem Vorbehandlungsschritt ausgewählt aus mindestens teilweiser Abtrennung von anderen Kunststoffen, bevorzugt durch Aussortierung, chemischem Vorbehandlungsschritt, Zerkleinerungsschritt unterworfen, bevor es in Schritt (b) eingesetzt wird.

In den Fällen, in denen das **PET** in Mischung mit anderen Kunststoffen vorliegt, wird das **PET** bevorzugter erst mindestens teilweise von anderen Kunststoffen abgetrennt, dann mindestens einmal chemisch vorbehandelt und schließlich zerkleinert.

Bei dem chemischen Vorbehandlungsschritt handelt es sich insbesondere um einen Waschschritt. Ein solcher Waschschritt hat den Vorteil, dass vor der Durchführung des Schrittes (b) eventuelle Verunreinigungen, insbesondere Speisereste, Reste von Kosmetika und/oder Körperflüssigkeiten (z.B. Blut, Sperma, Fäkalien), entfernt werden. Solche Verunreinigungen könnten die Effizienz der Umsetzung in Schritt (b) herabsetzen und/oder die Reinheit des damit erhaltenen **BHET** verschlechtern.

Beim chemischen Vorbehandlungsschritt, insbesondere dem Waschschritt, wird der Abfall insbesondere in einer Waschlösung bei einer Temperatur im Bereich von 30 °C bis 99 °C, bevorzugt im Bereich von 50 C° bis 90 °C, noch bevorzugter im Bereich von 70 °C bis 85 °C, erhitzt.

Typische Waschlösungen sind dem Fachmann geläufig und bevorzugt ausgewählt aus:
- wässrige Lösung eines Tensids, bevorzugt eines nicht-ionischen Tensids;
- wässrige Lösung eines Alkalimetallhydroxids oder Erdalkalimetallhydroxids; bevorzugt wässrige NaOH.

Die Behandlungszeit des chemischen Vorbehandlungsschritts, insbesondere des Waschschritts, beträgt dabei insbesondere im Bereich von 1 min bis 12 h, bevorzugt im Bereich von 10 min bis 6 h, bevorzugter im Bereich von 30 min bis 2 h, noch bevorzugter im Bereich von 45 bis 90 min, am bevorzugtesten bei 60 min.

Nach der Behandlung des **PET** mit dem chemischen Vorbehandlungsschritt, insbesondere dem Waschschritt, wird die wässrige Lösung abgetrennt, z.B. durch Filtration, und das gesäuberte **PET** bevorzugt mindestens einmal mit Wasser gewaschen, um Reste der Waschlösung zu entfernen. Der so erhaltene **PET**-Abfall wird dann getrocknet, insbesondere in einem Trockenschrank.

Die zum Trocknen eingesetzt Temperatur liegt dabei insbesondere im Bereich von 30 °C bis 120 °C, bevorzugt im Bereich von 50 °C bis 100 °C, bevorzugter im Bereich von 60 °C bis 90 °C, am bevorzugtesten bei 80 °C.

Der Zerkleinerungsschritt hat den Vorteil, dass die in Schritt (b) für die Reaktion zur Verfügung stehende Oberfläche des **PET** erhöht wird. Dadurch erhöht sich die Reaktionsgeschwindigkeit der Umsetzung in Schritt (b). Die Zerkleinerung kann in den Fachmann bekannten Apparaturen erfolgen, beispielsweise einem *Shredder* oder einer Schneidmühle.

In einer weiteren Ausführungsform des erfindungsgemäßen Verfahrens wird das **PET,** bevor es dem Schritt (b) unterworfen wird, entfärbt oder gezielt gefärbt. Dies kann mit dem Fachmann bekannten Verfahren durchgeführt werden, z.B. Entfärbung mit Wasserstoffperoxid oder Färbung mit einem Farbstoff.

### 4.2 Umsetzungsbedingungen in Schritt (b)

In Schritt (b) des erfindungsgemäßen Verfahrens wird **PET** in einer Mischung mit Glykol und mindestens einem Teil des in Schritt (a) erhaltenen M_{A}OR zu **BHET** umgesetzt.

Es versteht sich von selbst, dass **"PET** wird in einer Mischung umfassend Glykol und mindestens einen Teil des in Schritt (a) erhaltenen M_{A}OR zu Bis-(2-hydroxyethyl)-terephthalat **BHET** umgesetzt" bedeutet, dass Schritt (b) in einer Mischung umfassend **PET,** Glykol und mindestens einen Teil des in Schritt (a) erhaltenen M_{A}OR durchgeführt wird. In der Umsetzung gemäß Schritt (b) wird **PET** formal an den intramolekularen Esterbindungen [siehe nachfolgend gezeigte Struktur (Ξ)] durch Glykol umgeestert, wobei das Alkoholatanion von M_{A}OR als Katalysator fungiert.

Ohne durch eine bestimmte Theorie gebunden zu sein, umfasst der Mechanismus der Spaltung des **PET zu BHET** zunächst den nukleophilen Angriff des Alkoholatanions RO⁻ auf die Esterbindung und Spaltung des Polymers **PET,** wodurch sich intermediär der Ester der Terephthalsäureeinheit mit dem Alkohol ROH bildet, gefolgt von der Umesterung dieses Esters mit Glykol. Dies ist im Folgenden anhand einer Esterbindung des **PET** schematisch dargestellt:

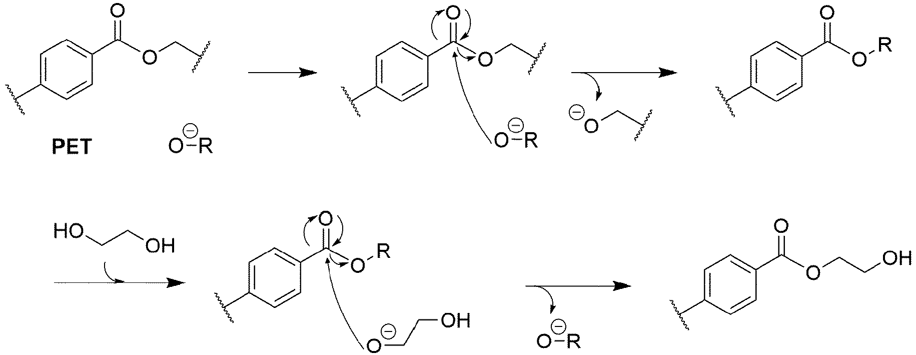

Der Schritt (b) des erfindungsgemäßen Verfahrens kann dabei auf jede dem Fachmann geläufige Art und Weise durchgeführt werden. Typischerweise werden in Schritt (b) die Komponenten **PET,** Glykol, das in Schritt (a) erhaltene M_{A}OR in beliebiger Reihenfolge vermischt und die Umsetzungsbedingungen eingestellt, wodurch **PET** gemäß Schritt (b) zu **BHET** gespalten wird.

Insbesondere wird in Schritt (b) **PET** mit Glykol und mindestens einem Teil des in Schritt (a) erhaltenen M_{A}OR, welches in einer bevorzugten Ausführungsform des Schrittes (a) in Form der Lösung **S_{AP}** oder der Lösung **S_{AP}*** oder als Feststoff **F*** erhalten wird, zu einer Mischung **M₁** umfassend **PET,** Glykol und M_{A}OR vermischt und **PET** in der Mischung **M₁** mit Glykol und M_{A}OR mindestens teilweise zu Bis-(2-hydroxyethyl)-terephthalat **BHET** umgesetzt. Bevorzugt wird dadurch nach Beendigung des Schrittes (b) eine Mischung **M₂** erhalten, welche **BHET** enthält, und welche insbesondere zusätzlich Glykol, M_{A}OR und gegebenenfalls nicht umgesetztes **PET** sowie gegebenenfalls **MHET** und gegebenenfalls **TS** umfasst.

In einer bevorzugten Ausführungsform des Schrittes (b) werden eine oder zwei der drei Komponenten, welche aus **PET,** Glykol, das in Schritt (a) erhaltene M_{A}OR ausgewählt sind, vorgelegt, die Reaktionsbedingungen darin eingestellt, und schließlich dann die übrige/ übrigen Komponente/ Komponenten, welche aus **PET**, Glykol, das in Schritt (a) erhaltene M_{A}OR ausgewählt sind, zugegeben. Unmittelbar nach der Zugabe dieser letzten Komponente wird dann die Mischung **M₁** erhalten, in welcher, da die Reaktionsbedingungen schon eingestellt sind, dann unmittelbar **PET** gemäß Schritt (b) zu **BHET** gespalten wird, und am Ende des Schrittes (b) dann die Mischung **M₂** erhalten wird, welche **BHET** enthält, und welche insbesondere zusätzlich Glykol, M_{A}OR und gegebenenfalls nicht umgesetztes **PET** sowie gegebenenfalls **MHET** und gegebenenfalls **TS** umfasst.

In einer weiteren alternativen Ausführungsform des Schrittes (b), welche insbesondere bei einer kontinuierlichen Verfahrensführung durchgeführt wird, wird mindestens eine, bevorzugt zwei, bevorzugt alle drei der Komponenten **PET**, Glykol, das in Schritt (a) erhaltene M_{A}OR, einer Mischung M₁ zugeführt, welche **PET**, Glykol, das in Schritt (a) erhaltene M_{A}OR und **BHET** umfasst, d.h. das in dieser Mischung M₁ die Umsetzung von **PET** gemäß Schritt (b) zu **BHET** während der Zugabe mindestens einer der drei Komponenten **PET**, Glykol, das in Schritt (a) erhaltene M_{A}OR abläuft. Bei Zugabe von mindestens zweien der Komponenten **PET**, Glykol, das in Schritt (a) erhaltene M_{A}OR zu dieser Mischung **M₁** werden diese insbesondere getrennt voneinander zugegeben. Bevorzugt wird dadurch nach Beendigung des Schrittes (b) eine Mischung **M₂** erhalten, welche **BHET** enthält, und welche insbesondere zusätzlich Glykol, M_{A}OR und gegebenenfalls nicht umgesetztes **PET** sowie gegebenenfalls **MHET** und gegebenenfalls **TS** umfasst.

In der erfindungsgemäß bevorzugten Ausführungsform des Schrittes (a), in welcher ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt wird, wobei am unteren Ende von **RR_{A}** ein Sumpfproduktstrom **S_{AP}** umfassend M_{A}OR und ROH entnommen wird und wobei am oberen Ende von **RR_{A}** ein Brüdenstrom **S_{AB}** umfassend Wasser und gegebenenfalls ROH entnommen wird,
und in welcher im optionalen Schritt (a*) ROH mindestens teilweise aus **S_{AP}** entfernt wird, so dass M_{A}OR als Feststoff **F*** oder als Lösung **S_{AP}*** umfassend M_{A}OR und ROH, wobei **S_{AP}*** einen gegenüber **S_{AP}** verringerten Massenanteil an ROH aufweist, erhalten wird,
wird dabei in Schritt (b) insbesondere **PET** in einer Mischung umfassend Glykol und mindestens einen Teil des von **S_{AP}** umfassten M_{A}OR oder, falls Schritt (a*) durchgeführt wird, mindestens einem Teil des von **F*** umfassten M_{A}OR oder mindestens einem Teil des von **S_{AP}*** umfassten M_{A}OR zu Bis-(2-hydroxyethyl)-terephthalat **BHET** umgesetzt .

"Mindestens einen Teil des von **S_{AP}** umfassten M_{A}OR oder, falls Schritt (a*) durchgeführt wird, mindestens einem Teil des von **F*** umfassten M_{A}OR oder mindestens einem Teil des von **S_{AP}*** umfassten M_{A}OR " impliziert, dass **S_{AP}** umfassend M_{A}OR bzw. **F*** umfassend M_{A}OR bzw. **S_{AP}*** umfassend M_{A}OR zugegeben wird. In dieser besonderen Ausführungsform wird demnach insbesondere eine Mischung umfassend **PET,** Glykol und **S_{AP}** umfassend M_{A}OR oder, wenn Schritt (a*) durchgeführt wird, **F*** umfassend M_{A}OR oder S_{AP}* umfassend M_{A}OR hergestellt und **PET** dann damit und mit Glykol gemäß Schritt (b) zu **BHET** umgesetzt.

Die Umsetzung in Schritt (b) wird dabei insbesondere bei einer Temperatur von mindestens 100 °C, bevorzugt bei einer Temperatur im Bereich von 100 °C bis 197 °C, bevorzugter bei einer Temperatur im Bereich von 130 °C bis 197 °C, bevorzugter bei einer Temperatur im Bereich von 150 °C bis 197 °C, bevorzugter bei einer Temperatur im Bereich von 175 °C bis 197 °C durchgeführt.

Die Umsetzung in Schritt (b) wird bevorzugt bei der Siedetemperatur des Glykols durchgeführt.

Noch bevorzugter wird Glykol dabei refluxiert, das heißt Glykol wird aus der Umsetzung verdampft, kondensiert und dann wieder in die Umsetzung rückgeführt. Diese Refluxierung kann mit dem Fachmann geläufigen Mitteln eingestellt werden, beispielsweise in einer Destillationsapparatur.

Diese Ausführungsform ist insbesondere dann vorteilhaft, wenn M_{A}OR als Lösung in ROH, also insbesondere in Form von **S_{AP}** oder **S_{AP}*,** in Schritt (b) zur Mischung zugegeben wird, da der überschüssige Alkohol ROH, der einen niedrigeren Siedepunkt als Glykol aufweist, dann aus der Mischung verdampft. Dies reduziert das Auftreten von Nebenprodukten zusätzlich.

Bevorzugt wird die Umsetzung in Schritt (b) so lange, d.h. bis zu einem Zeitpunkt **t_{b},** durchgeführt bis sich mindestens **P** = 10 %, bevorzugt mindestens **P** = 20 %, bevorzugter mindestens **P** = 25 %, bevorzugter mindestens **P** = 30 %, bevorzugter mindestens **P** = 40 %, bevorzugter mindestens **P** = 50 %, bevorzugter mindestens **P** = 60 %, bevorzugter mindestens **P** = 70 %, bevorzugter mindestens **P** = 80 %, bevorzugter mindestens **P** = 90 %, bevorzugter mindestens **P** = 95 %, noch bevorzugter mindestens **P** = 99 % des in Schritt (b) eingesetzten **PET**s umgesetzt haben.

Dieser prozentuale Anteil **P** wird nach der folgenden Formel berechnet: $P = \left({n}_{TS}+{n}_{MHET}+{n}_{BHET}\right) / {n}_{PET} .$

Dabei ist n**_{PET}** die Stoffmenge an Wiederholungseinheiten der folgenden Struktur (Ξ) in dem in Schritt (b) eingesetzten **PET:** 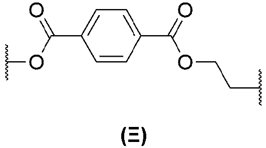
n**_{TS}** ist die Stoffmenge an **TS,** die sich von Beginn des Schrittes (b) bis zum Zeitpunkt t_{b} in Schritt (b) gebildet haben.
n**_{MHET}** ist die Stoffmenge an **MHET,** die sich von Beginn des Schrittes (b) bis zum Zeitpunkt t_{b} in Schritt (b) gebildet haben.
n**_{BHET}** ist die Stoffmenge an **BHET,** die sich von Beginn des Schrittes (b) bis zum Zeitpunkt t_{b} in Schritt (b) gebildet haben.

Die Strukturen der Verbindungen **BHET, MHET, TS** sind wie folgt: 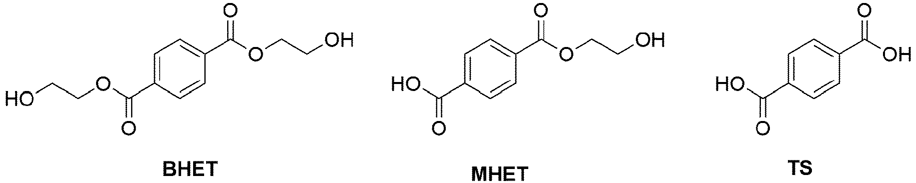 **"MHET"** umfasst auch das entsprechende Carboxylat der gezeigten Struktur. **"TS"** umfasst auch das entsprechende Mono- und Dicarboxylat der gezeigten Struktur.

In Schritt (b) wird insbesondere so viel M_{A}OR eingesetzt, dass das Gesamtgewicht des in Schritt (b) des erfindungsgemäßen Verfahrens eingesetzten M_{A}OR bezogen auf das Gesamtgewicht des in Schritt (b) des erfindungsgemäßen Verfahrens eingesetzten **PETs** im Bereich von 0.1 bis 100 Gew.-%, bevorzugt im Bereich von 0.5 bis 80 Gew.-%, bevorzugter im Bereich von 1.0 bis 50 Gew.-%, bevorzugter im Bereich von 1.5 bis 25 Gew.-%, bevorzugter im Bereich von 2.0 bis 10 Gew.-%, bevorzugter im Bereich von 2.5 bis 6.0 Gew.-%, besonders bevorzugt bei 3.5 bis 5.0 Gew-%, am bevorzugtesten bei 3.9 Gew.-% liegt.

Das Verhältnis des Gewichts [in kg] des in Schritt (b) des erfindungsgemäßen Verfahrens eingesetzten Glykols bezogen auf das Gewicht [in kg] des in Schritt (b) des erfindungsgemäßen Verfahrens eingesetzten **PETs** liegt insbesondere im Bereich von 1 : 1 bis 100 : 1, bevorzugt im Bereich von 2 : 1 bis 50 : 1, bevorzugter im Bereich von 3 : 1 bis 40 : 1, bevorzugter im Bereich von 4 : 1 bis 30 : 1, bevorzugter im Bereich von 5 : 1 bis 20 : 1, bevorzugter im Bereich von 6 : 1 bis 10 : 1, besonders bevorzugt im Bereich von 7 : 1 bis 9 : 1, am bevorzugtesten bei 8 : 1.

Die Umsetzung in Schritt (b) kann mit dem Fachmann geläufigen Geräten erfolgen.

Nach Beendigung des Schrittes (b) des erfindungsgemäßen Verfahrens liegt bevorzugt in der nach Schritt (b) erhaltenen Mischung das molare Verhältnis η der Stoffmenge von **BHET** (n_{BHET}) zur Summe der Stoffmengen von **MHET** und **TS** (n_{MHET} + n_{TS}) im Bereich 1 : 1 bis 1000 : 1, bevorzugt 2 : 1 bis 500 : 1, bevorzugter 4 : 1 bis 300 : 1, noch bevorzugter 10 : 1 bis 100 : 1, noch mehr **BHET** in Schritt (c) aus der nach Beendigung des Schrittes (b) erhaltenen Mischung abfiltriert und dann auskristallisiert.

### 5. Verfahren zum Recycling von PET

Das nach Beendigung des Schrittes (b) im erfindungsgemäßen Verfahren erhaltene **BHET** wird bevorzugt in einem Verfahren zum Recycling von Polyethylenterephthalat **PET** in einem Schritt (ζ) zu **PET** polymerisiert.

Diese Polymerisierung ist dem Fachmann als "Polykondensation" bekannt und z.B. in EP 0 723 951 A1 und von Th. Rieckmann und S. Völker in Kapitel 2 "Poly(Ethylen Terephthalate) Polymerization - Mechanism, Catalysis, Kinetics, Mass Transfer and Reactor Design" auf Seite 92 des Buches " Modern Polyesters: Chemistry and Technology of Polyesters and Copolyesters. Edited by J. Scheirs and T. E. Long, 2003, John Wiley & Sons, Ltd ISBN: 0-471-49856-4" beschrieben.

Insbesondere wird dazu **BHET** im Schritt (ζ) in Gegenwart von Katalysatoren, bei denen es sich insbesondere um Katalysatoren, die aus der Gruppe bestehend aus Antimonverbindungen, bevorzugt Sb₂O₃, ausgewählt sind, handelt, wieder zu **PET** polymerisiert.

Bevorzugt wird die Polymerisierung von **BHET zu PET** in Schritt (ζ) mindestens bei der Siedetemperatur des Glykols durchgeführt. Insbesondere wird während der Polymerisierung in Schritt (ζ) Glykol aus dem Reaktionsgemisch entfernt, um das Reaktionsgleichgewicht auf die Seite des Polymers **PET** zu verschieben.

Bevorzugter wird die Polymerisierung von **BHET zu PET** in Schritt (ζ) bei der Siedetemperatur des Glykols durchgeführt. Noch bevorzugter wird dann während der Polymerisierung in Schritt (ζ) Glykol aus dem Reaktionsgemisch entfernt, um das Reaktionsgleichgewicht auf die Seite des Polymers **PET** zu verschieben.

Dies wird insbesondere durch Destillation bei einem Druck < 0.1 MPa (1 bar), bevorzugt 10 Pa (0.1 mbar) bei der gleichzeitigen Siedetemperatur des Glykols beim jeweiligen Druck erreicht.

Dies wird insbesondere durch Destillation bei einem Druck < 1 bar, bevorzugt 0.1 mbar bei der gleichzeitigen Siedetemperatur des Glykols beim jeweiligen Druck erreicht.

### Beispiele

### 1. Erfinderisches Beispiel E1:

### Depolymerisierung von PET mit methanolischer Natriummethylatösung aus der Reaktivdestillation

30%-ige methanolische Natriummethylat-Lösung wurde durch Reaktivdestillation gemäß dem in Beispiel 1.1 der EP 1 997 794 A1 beschriebenen Verfahren erhalten.

150 g **PET** wurden dann in einem Autoklaven mit 1200 g Ethylenglykol vorgelegt. Die Lösung wurde anschließend unter Rühren auf 175 °C erhitzt. Sobald die Temperatur von 175 °C erreicht war, wurden 12.3 g (entsprechend 0.068 mol NaOCH₃) der durch die Reaktivdestillation erhaltenen 30%-igen methanolischen Natriummethylat-Lösung hinzugefügt. Die Reaktion wurde über zwölf Stunden durchgeführt und der Reaktoraustrag nach dem Abkühlen untersucht. Der erhaltene Umsatz wurde per Gaschromatographie bestimmt. Die Menge der Nebenkomponenten Mono-(2-hydroxyethyl)-terephthalsäure (= **"MHET")** (1) sowie Terephthalsäure (= "**TS"**) (2) in Bezug auf das entstehende Hauptprodukt **(BHET)** ist in der Abbildung dargestellt (in % in Bezug auf das erhaltene **BHET;** von links oben → rechts unten gestreifter Balken: "\\\\\\").

### 2. Vergleichsbeispiel V1:

### Depolymerisierung von PET mit herkömmlich hergestellter methanolischer Natriummethylatösung

In einem Vergleichsversuch wurden 150 g **PET** in einem Autoklaven mit 1200 g Ethylenglykol vorgelegt. Die Lösung wurde anschließend unter Rühren auf 175 °C erhitzt. Sobald die Temperatur von 175 °C erreicht war, wurden 2.7 g festes NaOH in 11 g Methanol (entsprechend 0.068 mol NaOCH₃) hinzugefügt. Die Reaktion wurde über fünf Stunden durchgeführt und der Reaktoraustrag nach dem Abkühlen untersucht. Die Menge der Nebenkomponenten Mono-(2-hydroxyethyl)-terephthalsäure (= **"MHET")** (1) sowie Terephthalsäure (= "**TS**") (2) in Bezug auf das entstehende Hauptprodukt (BHET) ist in der Abbildung dargestellt (Balken: "■").

### 3. Ergebnis

Der Vergleich des Gehalts an **BHET, MHET** und **TS** im depolymerisierten Produkt im erfinderischen Beispiel **E1** und dem Vergleichsbeispiel **V1** zeigt, dass die erhaltene Menge an **BHET** in beiden Versuchen in etwa gleich war. Bei der Depolymerisierung unter Einsatz der durch Reaktivdestillation erhaltenen methanolischen Natriummethylatlösung wurde jedoch ein geringerer Anteil an unerwünschte Nebenprodukt **MHET,** bezogen auf **BHET,** erhalten. Zusätzlich konnte im erfindungsgemäßen Verfahren kein **TS** gefunden werden konnte, während der Anteil an diesem unerwünschten Nebenprodukt in der Reaktionsmischung aus **V1** hoch war.

Demnach konnte beim erfinderischen Vorgehen anteilig mehr Spaltprodukt **BHET** gewonnen wird, welches vorteilhaft direkt in einer Polykondensation zu neuem Produkt **PET** umgesetzt werden kann.

## Patentansprüche

1. Verfahren zur Depolymerisierung von Polyethylenterephthalat **PET,** umfassend die folgenden Schritte:
(a) M_{A}OH und ROH werden in einer Reaktivdestillation zu M_{A}OR umgesetzt, wobei M_{A} ein Alkalimetall ausgewählt aus Natrium, Kalium, Lithium ist, und wobei R ein Alkylrest mit 1 bis 6 Kohlenstoffatomen ist,
(b) **PET** wird in einer Mischung umfassend Glykol und mindestens einen Teil des in Schritt (a) erhaltenen M_{A}OR zu Bis-(2-hydroxyethyl)-terephthalat **BHET** umgesetzt.

2. Verfahren nach Anspruch 1, wobei in Schritt (a) ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohprodukt **RP_{A}** umfassend M_{A}OR, Wasser, ROH, M_{A}OH umgesetzt wird,
wobei am unteren Ende von **RR_{A}** ein Sumpfproduktstrom **S_{AP}** umfassend M_{A}OR und ROH entnommen wird und wobei am oberen Ende von **RR_{A}** ein Brüdenstrom **S_{AB}** umfassend Wasser und gegebenenfalls ROH entnommen wird,
und wobei in einem optionalen Schritt (a*) ROH mindestens teilweise aus **S_{AP}** entfernt wird, so dass M_{A}OR als Feststoff **F*** oder als Lösung **S_{AP}*** umfassend M_{A}OR und ROH, wobei **S_{AP}*** einen gegenüber **S_{AP}** verringerten Massenanteil an ROH aufweist, erhalten wird,
und wobei in Schritt (b) **PET** in einer Mischung umfassend Glykol und mindestens einen Teil des von **S_{AP}** umfassten M_{A}OR oder, falls Schritt (a*) durchgeführt wird, mindestens einen Teil des von **F*** umfassten M_{A}OR oder mindestens einen Teil des von **S_{AP}*** umfassten M_{A}OR zu Bis-(2-hydroxyethyl)-terephthalat **BHET** umgesetzt wird.

3. Verfahren nach Anspruch 2, wobei **S_{AB}** Wasser und ROH umfasst, und diese in einer Rektifikationskolonne **RD_{A}** mindestens teilweise voneinander getrennt werden.

4. Verfahren nach Anspruch 2 oder 3, wobei der Gehalt von Wasser in **S_{AP}** oder, falls Schritt (a*) durchgeführt wird, in **S_{AP}*** bzw. **F*** bei < 1 Gew.-% liegt.

5. Verfahren nach einem der Ansprüche 1 bis 4, wobei R aus Methyl, Ethyl ausgewählt ist.

6. Verfahren nach einem der Ansprüche 1 bis 5, wobei M_{A} ein Alkalimetall ausgewählt aus Natrium, Kalium ist.

7. Verfahren nach einem der Ansprüche 1 bis 6, wobei Schritt (b) so lange durchgeführt wird, bis sich mindestens **P** = 10 % des in Schritt (b) eingesetzten **PET**s umgesetzt haben.

8. Verfahren nach einem der Ansprüche 1 bis 7, wobei in Schritt (b) so viel M_{A}OR eingesetzt wird, dass das Gesamtgewicht des in Schritt (b) eingesetzten M_{A}OR bezogen auf das Gesamtgewicht des in Schritt (b) eingesetzten **PET**s im Bereich von 0.1 bis 100 Gew.-% liegt.

9. Verfahren nach einem der Ansprüche 1 bis 8, wobei **BHET** in einem weiteren Schritt (c) mindestens teilweise aus der nach Beendigung des Schrittes (b) erhaltenen Mischung abgetrennt wird.

10. Verfahren nach Anspruch 9, wobei die Abtrennung von **BHET** durch Kristallisation und/oder Destillation erfolgt.

11. Verfahren nach einem der Ansprüche 1 bis 10, wobei **PET** mindestens einem Vorbehandlungsschritt ausgewählt aus chemischem Vorbehandlungsschritt, Zerkleinerungsschritt unterworfen wird, bevor es in Schritt (b) eingesetzt wird.

12. Verfahren zum Recycling von Polyethylenterephthalat **PET,** in welchem mit einem Verfahren nach einem der Ansprüche 1 bis 11 **BHET** erhalten wird und in einem Schritt (ζ) das so erhaltene **BHET zu PET** polymerisiert wird.

13. Verfahren nach Anspruch 12, wobei die Polymerisierung von **BHET zu PET** in Schritt (ζ) bei mindestens der Siedetemperatur des Glykols durchgeführt wird.

14. Verfahren nach Anspruch 12 oder 13, wobei die Polymerisierung in Schritt (ζ) in Gegenwart eines Katalysators durchgeführt wird.

15. Verfahren nach Anspruch 14, wobei der Katalysator aus der Gruppe bestehend aus Antimonverbindungen ausgewählt ist.

## Claims

1. Method of depolymerization of polyethylene terephthalate PET, comprising the following steps:
(A) M_{A}OH and ROH are converted to M_{A}OR in a reactive distillation, where M_{A} is an alkali metal selected from sodium, potassium, lithium, and where R is an alkyl radical having 1 to 6 carbon atoms,
(b) PET is converted to bis(2-hydroxyethyl) terephthalate BHET in a mixture comprising glycol and at least a portion of the M_{A}OR obtained in step (a).

2. Method according to Claim 1, wherein, in step (a), a reactant stream S_{AE1} comprising ROH is reacted with a reactant stream S_{AE2} comprising M_{A}OH in countercurrent in a reactive rectification column RR_{A} to give a crude product RP_{A} comprising M_{A}OR, water, ROH, M_{A}OH,
wherein a bottom product stream S_{AP} comprising M_{A}OR and ROH is withdrawn at the lower end of RR_{A} and wherein a vapour stream S_{AB} comprising water, with or without ROH, is withdrawn at the upper end of RR_{A},
and wherein, in an optional step (a*), ROH is removed at least partly from S_{AP}, such that M_{A}OR is obtained in solid form F* or as a solution S_{AP}* comprising M_{A}OR and ROH, where S_{AP}* has a reduced proportion by mass of ROH compared to S_{AP},
and wherein, in step (b), PET is converted to bis(2-hydroxyethyl) terephthalate BHET in a mixture comprising glycol and at least a portion of the M_{A}OR encompassed by S_{AP} or, if step (a*) is conducted, at least a portion of the M_{A}OR encompassed by F* or at least a portion of the M_{A}OR encompassed by S_{AP}*.

3. Method according to Claim 2, wherein S_{AB} comprises water and ROH, and these are at least partly separated from one another in a rectification column RD_{A}.

4. Method according to Claim 2 or 3, wherein the content of water in S_{AP} or, if step (a*) is carried out, in S_{AP}* or F* is < 1% by weight.

5. Method according to any of Claims 1 to 4, wherein R is selected from methyl, ethyl.

6. Method according to any of Claims 1 to 5, wherein M_{A} is an alkali metal selected from sodium, potassium.

7. Method according to any of Claims 1 to 6, wherein step (b) is conducted until at least P = 10% of the PET used in step (b) has been converted.

8. Method according to any of Claims 1 to 7, wherein a sufficient amount of M_{A}OR is used in step (b) that the total weight of the M_{A}OR used in step (b), based on the total weight of the PET used in step (b), is within a range from 0.1% to 100% by weight.

9. Method according to any of Claims 1 to 8, wherein BHET, in a further step (c), is at least partly separated from the mixture obtained after step (b) has ended.

10. Method according to Claim 9, wherein the separation of BHET is effected by crystallization and/or distillation.

11. Method according to any of Claims 1 to 10, wherein PET is subjected to at least one pretreatment step selected from chemical pretreatment step, comminution step, before being used in step (b).

12. Method of recycling polyethylene terephthalate PET, in which BHET is obtained by a method according to any of Claims 1 to 11 and the BHET thus obtained is polymerized to PET in a step (ζ).

13. Method according to Claim 12, wherein the polymerization of BHET to PET in step (ζ) is conducted at at least the boiling temperature of the glycol.

14. Method according to Claim 12 or 13, wherein the polymerization in step (ζ) is conducted in the presence of a catalyst.

15. Method according to Claim 14, wherein the catalyst is selected from the group consisting of antimony compounds.

## Revendications

1. Procédé de dépolymérisation de poly(téréphtalate d'éthylène) PET, comprenant les étapes suivantes :
(a) du M_{A}OH et du ROH sont transformés dans une distillation réactive en M_{A}OR, M_{A} représentant un métal alcalin choisi parmi le sodium, le potassium, le lithium et R représentant un radical alkyle comprenant 1 à 6 atomes,
(b) du PET est transformé, dans un mélange comprenant du glycol et au moins une partie du M_{A}OR obtenu à l'étape (a), en téréphtalate de bis-(2-hydroxyethyle) BHET.

2. Procédé selon la revendication 1, dans lequel, dans l'étape (a), un flux de départ S_{AE1} comprenant du ROH est transformé avec un flux de départ S_{AE2} comprenant du M_{A}OH à contre-courant dans une colonne de rectification réactive RR_{A} en un produit brut RP_{A} comprenant du M_{A}OR, de l'eau, du ROH, du M_{A}OH,
dans lequel, en l'extrémité inférieure de RR_{A}, est prélevé un flux de produit de fond S_{AP} comprenant du M_{A}OR et du ROH et, en l'extrémité supérieure de RR_{A}, est prélevé un flux de buées S_{AB} comprenant de l'eau et le cas échéant du ROH,
et dans lequel, dans une étape facultative (a*), du ROH est éliminé au moins partiellement de S_{AP}, de telle sorte que du M_{A}OR est obtenu sous la forme d'un solide F* ou sous la forme d'une solution S_{AP}* comprenant du M_{A}OR et du ROH, S_{AP}* présentant une fraction massique de ROH réduite par rapport à celle de S_{AP},
et dans lequel, dans l'étape (b), du PET, dans un mélange comprenant du glycol et au moins une partie du M_{A}OR compris dans S_{AP} ou, si l'étape (a*) est mise en œuvre, au moins une partie du M_{A}OR compris dans F* ou au moins une partie du M_{A}OR compris dans S_{AP}*, est transformé en téréphtalate de bis-(2-hydroxyéthyle) BHET.

3. Procédé selon la revendication 2, dans lequel S_{ab} comprend de l'eau et du ROH et ceux-ci sont séparés au moins partiellement l'un de l'autre dans une colonne de rectification RD_{A}.

4. Procédé selon la revendication 2 ou 3, dans lequel la teneur en eau dans S_{AP} ou, si l'étape (a*) est mise en œuvre, dans S_{AP}* ou, selon le cas, F* se situe à < 1% en poids.

5. Procédé selon l'une des revendications 1 à 4, dans lequel R est choisi parmi méthyle et éthyle.

6. Procédé selon l'une des revendications 1 à 5, dans lequel M_{A} représente un métal alcalin choisi parmi sodium et potassium.

7. Procédé selon l'une des revendications 1 à 6, dans lequel l'étape (b) est mise en œuvre jusqu'à ce qu'au moins P = 10% du PET utilisé dans l'étape (b) soient transformés.

8. Procédé selon l'une des revendications 1 à 7, dans lequel, dans l'étape (b), on utilise une quantité de M_{A}OR telle que le poids total du M_{A}OR utilisé dans l'étape (b), par rapport au poids total du PET utilisé dans l'étape (b), se situe dans la plage de 0,1 à 100% en poids.

9. Procédé selon l'une des revendications 1 à 8, dans lequel le BHET est séparé au moins partiellement à partir du mélange obtenu après la fin de l'étape (b) dans une autre étape (c).

10. Procédé selon la revendication 9, dans lequel la séparation de BHET est mise en œuvre par cristallisation et/ou distillation.

11. Procédé selon l'une des revendications 1 à 10, dans lequel le PET est soumis à au moins une étape de prétraitement choisie parmi une étape de prétraitement chimique et une étape de broyage avant d'être utilisé dans l'étape (b).

12. Procédé de recyclage de poly(téréphtalate d'éthylène) PET, dans lequel, à l'aide d'un procédé selon l'une des revendications 1 à 11, du BHET est obtenu et, dans une étape (ζ), le BHET ainsi obtenu est polymérisé en PET.

13. Procédé selon la revendication 12, dans lequel la polymérisation de BHET en PET dans l'étape (ζ) est mise en œuvre à au moins la température d'ébullition du glycol.

14. Procédé selon la revendication 12 ou 13, dans lequel la polymérisation dans l'étape (ζ) est mise en œuvre en présence d'un catalyseur.

15. Procédé selon la revendication 14, dans lequel le catalyseur est choisi dans le groupe constitué par les composés d'antimoine.
